# EUROPEAN PATENT APPLICATION

(11) **EP 0 684 044 A2**
(43) Date of publication of application: **29.11.1995**
(21) Application number: 95201373.8
(22) Date of filing: 24.05.1995
(51) Int. Cl.: A61K 47/48

(54) **Composition consisting of a dendrimer and an active substance**

(30) Priority: 27.05.1994 NL 9400880; 11.11.1994 NL 9401886
(71) Applicant: DSM N.V., NL-6411 TE Heerlen (NL)
(72) Inventor: Jansen, Johan Franz Gradus Antonius, NL-5632 RW Eindhoven (NL); Meijer, Egbert Willem, NL-5583 GC Waalre (NL); De Brabander-van den Berg, Ellen Marleen Monique, NL-6438 BE Schinnen (NL)

(57) **Abstract**

The invention relates to a composition consisting of a dendrimer provided with blocking agents and an active substance occluded in the dendrimer.

According to the invention a blocking agent is a compound which is sterically of sufficient size, which readily enters into a chemical bond with the terminal groups of a dendrimer and which can also be split off from the dendrimer or be modified without thereby affecting the chemical structure of the dendrimer and the active substance. The blocking agent can be provided with protective groups.

Such conjugates have the advantage that the time at which the releasing of the active substance starts, can be controlled.

The invention also relates to a process for the preparation of such a composition and to a process for the controlled release of the active substance.

## Description

The invention relates to a composition consisting of a dendrimer and an active substance occluded in the dendrimer wherein the dendrimer has terminal groups.

Dendrimers are three-dimensional, highly branched oligomers and polymer molecules having a well defined chemical structure. Dendrimers in general comprise a core, a number of generations of branches and terminal groups. The generations of branched are composed of repeating structural units which extend outwardly from the dendrimer core. The terminal groups of a dendrimer of the N^{th} generation are the functional groups of the N^{th} (final) generation.

Between the branches there are cavities. The shape and the dimensions of these cavities vary depending on the generation and the chemical composition and structure of the repeating structural units. During the preparation of the dendrimer the degree of branching and the shape and dimensions of the cavities between the branches can be influenced. This can be achieved for instance by varying the repeating structural units, by increasing or decreasing the degree of branching or by introducing discontinuities into the branches.

From EP-B-271180 it is known to use dendrimers as a carrier for agricultural, pharmaceutical and other materials. Such carried materials may be associated with the dendrimers in various ways. For instance, the carried material may be contained in the cavities of the dendrimer. Dendrimers of the higher generations have a large number of terminal groups which are spatially so close to each other that they form a molecular barrier, so that release of the carried material from the dendrimer is diffusion controlled.

The drawback of such compositions known from the art, is that the external surface of the dendrimer is insufficiently close, as a result of which the time at which the release of the carried material starts cannot be controlled. The conjugates known from the art have the additional disadvantage that the carried material is not occluded in the dendrimer and can be washed out by washing the conjugate with a solvent and that there is virtually permanent diffusion of the carried material out of the dendrimer. Such compositions further have the drawback that the carried material is not necessarily within the internal structure of the dendrimer, but may be present on the surface of the dendrimer.

The object of the present invention is to provide a composition in which the active substance is occluded in the dendrimer and the time of the start of its release and the duration of the release can be controlled.

This is achieved according to the invention in that the terminal groups of the dendrimer are provided with blocking agents.

The composition according to the invention offers the advantage that undesired compounds which are not occluded in the dendrimer, but are present for instance on the surface of the dendrimer, can readily be removed for instance by washing or dialysis.

In the composition according to the invention the active substance is not chemically bonded to the dendrimer. The active substance according to the invention is in essence occluded physically in the dendrimer. This offers the advantage that the active substance and the dendrimer are not chemically modified. In the composition according to the invention, the active substance is taken in and retained in the internal structure of the dendritic macromolecule rather than on the external surface.

According to the invention, the molecule of the active substance may be occluded completely or partly. In the latter case part of the molecule is occluded while the other part extends outwardly from the dendritic macromolecule.

From relaxation time measurements by means of NMR spectroscopy it has surprisingly been found that the surface layer consisting of the blocking agents has solid state properties.

Surprisingly, it has also been found that sometimes the active substance is stabilized through occlusion in the dendrimer according to the invention. For example it appears that colouring agents show a higher resistance to light after occlusion in a dendrimer. The lifetime of radicals appears to be considerably prolonged after occlusion in a dendrimer.

According to the invention a blocking agent is a compound which sterically is of sufficient size, which readily enters into a chemical bond with the terminal groups of the dendrimer and which can also be split off from the dendrimer or be modified without thereby affecting the chemical structure of the dendrimer (e.g. the molecular architecture) and the active substance.

The blocking agent can be bound to the dendrimer in different ways, for example covalently, via hydrogen bridges or ionic bonding.

Suitable blocking agents according to the invention are compounds which can be split off from the dendrimer or can be modified for instance by means of a chemical reaction, hydrolysis in a neutral, acidic or basic medium, hydrogenation, reaction under influence of heat, photochemical reaction, a reaction in the presence of fluoride, a retro Michael reaction.

Suitable blocking agents according to the invention are for instance branched or non-branched Michael acceptors, active esters, optically active or non-active amino acids, nucleic acids, saccharides, isocyanates, aziridines, acid chlorides, anhydrides, aldehydes, ketones, acrylates, chiral epoxides, lactones, bislactide, fatty acids with 10-24 carbon atoms and polymers.

Examples of suitable activated esters, i.e. esters which contain an activated carbonyl group, are esters which also comprise an ether and/or thiol group. Examples of suitable polymeric blocking agents are polyethene, polypropene, nylon 4,6 and nylon 6. Examples of suitable Michael acceptors include isothyocyanates, sulphonychlorides, phosphonyl chlorides, polyethyleneglycol-4-nonyl-phenylether acrylate, polyethyleneglycol-phenylether acrylate, polyethylene glycol-phenylether methacrylate.

A particularly useful class of blocking agents according to the invention are amino acids, such as for example glycine, alanine, valine, leucine, isoleucine, phenylalanine, tyrosine, tryptophane, serine, threonine, methionine, cystine, proline, hydroxyproline, aspartic acid, glutamic acid, lysine, arginine, histidine.

The blocking agents according to the invention may also be provided with one or more protective groups which may be the same or different. Suitable protective groups according to the invention can readily split off from the blocking agent without thereby breaking up the chemical bond between the blocking agent and the dendrimer and without thereby affecting the chemical structure of the dendrimer and the active substance.

The blocking agents according to the invention may be provided with protective groups partly or completely.

Suitable protective groups according to the invention are described for instance in T.W. Green, Protective Groups in Organic Synthesis, John Wiley & Son, New York, 1981.

Suitable protective groups according to the invention are for instance hydrolyzable esters, ethers, thiol groups, sulphonic acid groups, trityl, silyl, t-butoxycarbonyl (BOC), 9-fluorenylmethylcarbamate (FMOC), 2,7-Di-t-butyl-[9-(10,10-dioxo-10,10,10,10-tetrahydrothioxanthyl)] methylcarbamate (DBD-TMOC), benzyloxycarbonyl (Z), trimethylsilylethoxycarbonyl (TEOC), adamantyloxycarbonyl (AdMOC), 1,1,4,4-tetramethyldisilylaza-cyclopentene (STABASE), benzoSTABASE, benzyl and t-butyl groups. A very suitable protective group is the BOC group.

The protective groups can be modified and/or split off from the blocking agent for instance by means of a chemical reaction, hydrolysis in a neutral, acidic or basic medium, hydrogenation, reaction under influence of heat, photochemical reaction, reaction in the presence of fluoride, retro Michael reaction.

According to the invention, modification for example also means effecting physical modifications such as for example changes of the molecular volume of the dendrimer and/or conformational changes, for example under influence of temperature or solvent. As a consequence of these physical modifications, all or part of the active substance(s) occlued in the dendrimer can be released.

Suitable blocking agents provided with protective groups according to the invention are for instance methyloxyfuranone, aziridines provided with a sulphonic acid or BOC group, acrylates provided with large ester groups and amino acids provided with one or more BOC groups. Very suitable blocking agents provided with protective groups are amino acids provided with one or more BOC groups.

In general the choice of a particular blocking agent, protective group or blocking agent provided with a protective group, will be determined by the person skilled in the art on the basis of the nature and generation of the dendrimer applied and the size of the molecules of the active substance to be occluded.

The number of terminal groups of the dendrimer that is provided with a blocking agent according to the invention in general varies depending on the nature and generation of the dendrimer applied and the nature and dimensions of the blocking agent. In practice the number of terminal groups provided with a blocking agent is such that the external surface is regarded as essentially closed and diffusion of the active substance out of the dendrimer is virtually not possible.

Mostly at least 30% of the terminal groups or functional groups of the dendrimer are provided with a blocking agent, often at least 50%, at least 70% or at least 90%.

All dendrimers are in principle suitable for application according to the invention. Examples of suitable dendrimers are described in Angew. Chem. Int. Ed. Engl. 29 (1990), pp. 138-175. This publication describes, inter alia, polyamidoamine (PAMAM) dendrimers, the core of which is an amine or ammonia and the branches of which are composed of, for instance, repeating 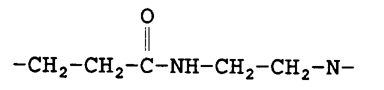
units.

Preferably in the composition according to the invention, dendrimers as described in WO-A-9314147 and WO-A-9502008 are used. The polypropylamine (POPAM) dendrimers described in WO-A-9314147 and WO-A-9502008 for instance have a core of diaminobutane. The branches may for instance be composed of successive, repeating -CH₂-CH₂-CH₂-N units. Such dendrimers offer the advantage of possessing a high thermal stability, being well soluble in a large number of organic solvents, possessing good stability in relation to hydrolysis and containing well accessible internal cavities and channels. The polypropylamine dendrimers described in WO-A-9314147 and WO-A-9502008 offer the additional advantage that a large number of generations can easily be prepared on a commercial scale.

According to the invention it is also possible to make use of dendrimers whose branches are prepared from halogen cyanide units comprising a monohalogenated hydrocarbon compound having 3-50 carbon atoms and at least 1 cyanide group, the halogen and the cyanide group being separated from each other by at least 3 carbon atoms.

Such dendrimers are prepared starting from a core molecule having at least one functional group acting as nucleophilic reactant in a nucleophilic substitution on a halogen cyanide. The functional group preferably is a primary or secondary amine group. In the preparation of such dendrimers
a) substantially each functional group of the core molecule is reacted with halogen cyanide units,
b) next, substantially each incorporated cyanide group is reduced to an amine,
c) after which substantially each amine group is reacted with halogen cyanide units.
Reaction steps b) and c) are repeated alternately until a dendritic macromolecule of the desired generation is obtained. The process can be stopped either after a step b) or a step c).

The halogen cyanide unit preferably contains 1-20 cyanide groups, the halogen preferably is chlorine or bromine.

Dendrimers according to the invention may also be wholly or partly modified in order to introduce a different functionality. This may be effected, for instance, by causing the terminal groups of the last generation to react, wholly or partly, optionally in the presence of a catalyst, with suitable reactants. Examples of such reactants are described for instance in WO-A-9314147 and WO-A-9502008.

Suitable dendrimers according to the invention are dendrimers with a perfectly branched structure as well as dendrimers with defects in the branching structure, dendrimers with an incomplete degree of branching (whereby the degree of branching is understood to be the ratio between the number of branches and the maximal possible number of branches), asymmetrically branched dendrimers, hyperbranched molecules, highly branched polymers and copolymers and/or blockcopolymers of highly branched and low branched polymers.

Dendrimers according to the invention are preferably symmetrically branched dendrimers.

According to the invention, mostly dendrimers of the higher generations are used, for example dendrimers of the third generation or higher, in particular dendrimers of the fourth generation or higher, more particular dendrimers of the fifth generation or higher.

Very suitable active substances according to the invention belong to the range of pharmaceutical compounds, drugs, agrochemicals -for example pesticides, herbicides, insecticides, fungants, pheromones, toxins-, materials used in personal care products, cosmetic products, food additives, additives for engineering plastics -such as for example stabilizers and flame resistant and/or flame retardant products-, chelating compounds, organic acids, non-metallic salts radicals, soaps, signal generators such as for example fluorescent and fosforescent compounds, signal absorbers such as for example UV-absorbing compounds, colourants, metals, metal compounds, radionuclides, radio active labelled compounds, D-II-A compounds, electron deficient and non-metallic electron rich compounds and precursors of the above mentioned compounds.

Suitable examples of pharmaceutical compounds are steroids such as for example precursors of vitamines; bile acids; hormones; sterols such as for example mestranol, estradiol, estrogen, estrone; antibiotics such as for example penicillin-V, azlocillin, tetracyclines; neurotransmittors and immunochemicals such as for example monoclonal and polyclonal antibodies, for example anti Ig, anti-H-Lectin, human IgA, IgG or IgM, and bovine IgG.

Suitable examples of agrochemicals include fertilizers such as for example acid phosphates, nitrophosphates and sulphates. Suitable examples of insecticides include chlorinated hydrocarbons, for example p-dichlorobenze; imidazoles and natural pyrethrins. Suitable examples of herbicides include carbamates, derivatives of phenol and derivatives of urea. Suitable examples of pheromones include synthetic and natural pheromones, for example 4-methyl-3-heptanone.

Suitable examples of food additives include flavours, fragrances and intensive sweeteners such as for example aspartame, saccharine, acesulfam-K, sucralose.

Suitable examples of organic acids and non-metallic salts include mono- and polycarboxylic acids such as saturated or unsaturated aliphatic acids and aromatic acids provided with substituents or not and salts thereof. Suitable examples of saturated aliphatic monocarboxylic acids include formic acid, acetic acid, propionic acid, n-butyric acid, isobutyric acid, n-valeric acid, caproic acid. Suitable examples of substituted aliphatic acids include glycolic acid, lactic acid, acrylic acid. Suitable examples of polycarboxylic acids include oxalic acid, malonic acid, succinic acid, glutaric acid, adipic acid, tartaric acid, maleic acid, fumaric acid and salts thereof. Representative aromatic acids include benzoic acid, phtalic acid, salycilic acid, anthranilic acid, cinnamic acid. Representative organic acids also include 3,5-dinitrobenzoic acid, 3,5-dimethoxybenzoic acid, 4-cyanobenzoic acid and anilinonaphtalenesulphonic acid. Suitable examples of non-metallic salts include the ammonium salt of anilinonaphtalenesulphonic acid.

Suitable examples of radicals or radical formers include hexamethylimidazolium-1-yloxy methylsulphate, diphenylpicrylhydrazyl, BPDA-complex (α,γ-bisdifenyleen-β-fenylallyl), TEMPO (2,2,6,6 tetramethyl-1-piperidinyloxy), doxyl (4,4-dimethyl-3-oxazolinyloxy-(4,4-dimethyloxazolidine-N-oxyl)), proxyl, proxylcarboxylic acid and derivatives thereof.

Suitable examples of signal absorbers include non-linear optical compounds, for example p-nitrodimethylaniline and dimethylaminonitrostilbene; UV absorbing compounds, for example azanaphtol compounds and crystalviolet; fluorescent compounds, for example eosine B, rhodamide B, fluoresceine and derivatives thereof.

Suitable examples of soaps include sulphate soaps; phosphoric acid soaps, for example diphenylphosphonic acid, phenylphosphonic acid; pyridine soaps for example dodecylpyridiniumchloride; alkaline soaps and heavy metal soaps for example aluminum, cobalt, calcium, zinc, lead soaps.

Suitable examples of electron deficient compounds include compounds that are electron deficient in the excited state and organic compounds provided with electron withdrawing groups conjugated or non-conjugated, for example carboxylic acid groups; amide groups; imide groups; cyano groups for example tricyanoethylene; sulphon groups; chinones and hydrochinones for example tetracyanohydrochinon.

Suitable examples of metals include metals from groups 1-14 and the Lanthanides and the Actinides of the Periodic System of the Elements as shown on the cover of the Handbook of Chemistry and Physics, 70th Edition. Representative exemplars of group 1 are Na, K, Rb, Cs; group 2: Mg, Ca; group 6: Cr, Mo; group 8:Fe, Ru; group 9: Co, Rh, Ir; group 10: Cu, Ag; group 12: Zn; Group 13 : Al; Group 14: Sn,Pb.

Suitable examples of precursors of for example pharmaceutical compounds and agrochemicals include benzoic acid, benzaldehyde, phenol, aliphatic and heterocyclic amines and derivatives thereof.

Depending on the shape and the dimensions of the molecules of the active substance, the person skilled in the art will select the most suitable generation of the dendrimer. In the case of POPAM dendrimers, dendrimers of generation 4 or higher are mostly applied.

The number of molecules of an active substance which can be occluded in a dendritic macromolecule depends on, among other things, the chemical composition and structure of the dendrimer, the shape and dimensions of the cavities in the dendrimer, the generation of the dendrimer and the shape and dimensions of the molecules of the active substance to be occluded.

According to the invention, one or more molecules of one or more active substances can be occluded in a dendritic macromolecule. The active substance may be divided statistically over the dendritic macromolecule or be concentrated in specific parts of the dendritic macromolecule.

The invention also relates to a process for the preparation of a composition comprising a dendrimer provided with blocking agents and an active substance occluded in the dendrimer.

M. Maciejewski, J. Macromol. Sci. Chem. A17 (4), pp. 689-703 (1982), describes that it is theoretically possible to occlude one or more guest molecules in a dendrimer if the preparation of the dendrimer is effected in the presence of guest molecules. At a specific generation such a number of terminal groups is present that the outer shell is blocked and is no longer permeable to guest molecules.

The drawback of such a process, however, is that guest molecules are occluded irreversibly in the dendrimer. For the guest molecules to be released it is necessary to remove one or more generations of the dendrimer, which entails the disintegration of the entire dendrimer structure.

In the process according to the invention an amount of the active substance to be occluded is added to a reaction mixture containing dendrimers. Simultaneously with the active substance or afterwards an amount of the blocking agent to be applied is added.

This process has the advantage that the composition can be washed or subjected to dialysis without loss of active substance occurring. This process has the additional advantage that any undesired substances present can be washed away without loss of the occluded active substance occurring. Preferably the blocking agent to be applied is added after the active substance.

The invention also relates to a process for the controlled release of the active substance.

This is achieved according to the invention by splitting off and/or modifying the blocking agent or part of the blocking agent.

By 'modifying the blocking agent' according to the invention is understood a modification of the physical interactions within the dendrimer provided with blocking agents, for example the physical interactions between and/or in the dendritic branches, the physical interactions between the blocking agents and the dendrimer or between the blocking agents themselves, and the physical interactions within the blocking agent as a result of which the occluded active substance is released. For example, by protonation of for example all or part of the amine groups of a polypropylamine dendrimer, the repulsion between the branches is increased and the occluded active substance is partly or completely released. Such a process has the advantage that the structure of the dendrimer with blocking agents remains intact and can be re-used.

According to a suitable embodiment of the invention the active substance is released by splitting off the protective group.

According to another embodiment of the invention the active substance is released by first splitting off the protective group and then splitting off the blocking agent.

Such a process offers the advantage that specific occluded active substances can be released in a controlled way. Splitting off the protective group will mostly cause smaller molecules of the active substance to be released. Splitting off the blocking agent will mostly cause the larger molecules of the active substance to be released. By making the right choice of, in particular, the size of the blocking agent and of the protective group, retarded release of the active substance, by splitting off the protective group, can be ensured for slow-release applications.

According to another suitable embodiment of the invention, the active substance is released by modification of the blocking agent, followed by splitting off the protective group and/or the blocking agent.

Suitable processes for splitting off and/or modifying the blocking agent according to the invention are for instance a chemical reaction, hydrolysis in a neutral, acidic or basic medium, hydrogenation, reaction under influence of heat, a photochemical reaction, a reaction in the presence of fluoride, retro Michael reactions. The blocking agent is preferably removed by hydrolysis in an acidic medium. Said processes are also suitable for splitting off a protective group or a blocking agent provided with one or more protective groups.

The invention will be further elucidated in the following non-restrictive examples.

NMR spectra were measured by means of a Bruker AM400 or a Varian Gemini 300 spectrometer in chloroform.

ESR spectra were measured by means of a Bruker ER 200D SRC spectrometer, using an X-ray band and a standard measurement cell. The temperature was measured by means of a Bruker ER 4111 thermometer. Low-temperature ESR spectra were measured in chloroform.

Infrared spectra were measured by means of a Perkin Elmer 1600 FT-IR spectrometer.

Specific rotations were determined by means of a JASCO DIP 370 polarimeter.

### Example I: Occlusion of 3,5-dinitrobenzoic acid.

200 mg of 3,5-dinitrobenzoic acid was added to a mixture of 35 mg (4.9 µmol) of a NH₂-terminated polypropylamine dendrimer of the fifth generation (64-cascade: 1,4-diaminobutane[4]:(1-azabutylidene)⁶⁰:propylamine) and 0.2 ml of triethylamine in 10 ml of dichloromethane. After stirring for 30 minutes 114 mg (0.3 mmol) of the hydroxysuccinimide ester of N-BOC-L-phenylalanine was added. After stirring for one night the reaction mixture was diluted to 50 ml with dichloromethane and next washed three times with 30 ml of water and three times with 30 ml of a saturated sodiumcarbonate solution. After drying in the presence of sodiumsulphate and evaporation of the remaining liquid, ¹H NMR analysis showed that 15-25 molecules of 3,5-dimethoxybenzoic acid were occluded in the dendrimer. About 80% of the dendrimer could be isolated.

### Example II: Occlusion of 3,5-dimethoxybenzoic acid.

200 mg of 3,5-dimethoxybenzoic acid was added to a mixture of 22 mg (3.1 µmol) of a NH₂-terminated polypropylamine dendrimer of the fifth generation (64-cascade: 1,4-diaminobutane[4]:(1-azabutylidene)⁶⁰:propylamine) and 0.2 ml of triethylamine in 10 ml of dichloromethane. After stirring for 30 minutes 72 mg (0.19 mmol) of the hydroxysuccinimide ester of N-BOC-L-phenylalanine was added. After stirring for one night the reaction mixture was diluted to 50 ml with dichloromethane and next washed three times with 30 ml of water and three times with 30 ml of a saturated sodiumcarbonate solution. After drying in the presence of sodiumsulphate and evaporation of the remaining liquid, ¹H NMR analysis showed that 15-25 molecules of 3,5-dimethoxybenzoic acid were occluded in the dendrimer. It was possible to isolate about 85% of the dendrimer.

### Example III: Occlusion and release of proxylcarboxylic acid (radical).

8 mg of proxylcarboxylic acid was added to a mixture of 59 mg of a NH₂-terminated polypropylamine dendrimer of the fifth generation (64-cascade: 1,4-diaminobutane[4]:(1-azabutylidene)⁶⁰:propylamine) and 0.4 ml of triethylamine in 10 ml of dichloromethane. After stirring for 30 minutes at room temperature 191 mg (0.53 mmol) of the hydroxysuccinimide ester of N-BOC-L-phenylalanine was added. After stirring for one night the reaction mixture was diluted to 50 ml with dichloromethane and next washed three times with 30 ml of water and three times with 30 ml of a saturated sodiumcarbonate solution. After drying in the presence of sodiumsulphate and evaporation of the remaining liquid, the yield was about 70%. By means of ESR spectroscopy it was shown that a few proxylcarboxylic acid radicals were occluded in the dendrimer.

Next, the dendrimer containing proxyl radicals was washed 16 times with 50 ml of a saturated sodium carbonate solution. Since the intensity of the signal corresponding to the radical was unchanged after washing, it may be assumed that the radical is occluded in the dendrimer. From the comparison with the spectrum of the free radical it was concluded that about 1 radical per dendrimer molecule was occluded.

Next, 34 mg of the radical-containing dendrimer was dissolved in 5 ml of dichloromethane, to which 1 ml of concentrated HCl was added. After stirring for about 60 hours 4 ml of water was added. The acid water layer was extracted by means of 5 portions of 5 ml of dichloromethane, dried over sodiumsulphate and boiled down, after which 5 ml of dichloromethane was added for measurement of the ESR spectrum. By means of ESR the presence of proxyl radicals in the dichloromethane solution was demonstrated.

After the radical-containing dichloromethane solution had been washed with a saturated sodiumcarbonate solution, no free proxyl radicals were detected in the dichloromethane layer. This implies that only the radicals released out of the dendrimer were extracted by means of sodiumcarbonate. It also means that sodiumcarbonate is not able to extract the radicals occluded in the dendrimer.

### Experiment IV: Blank experiment A.

By means of this experiment it is shown that the proxyl radical is not bonded chemically to the dendrimer.

For that purpose 10 mg of proxylcarboxylic acid was added to a solution of 0.5 ml of dicloromethane and 5 mg of a NH₂-terminated polypropylamine dendrimer of the fifth generation (64-cascade: 1,4-diaminobutane[4]:(1-azabutylidene)⁶⁰:propylamine), provided with BOC-terminated L-phenylalanine. The mixture was stirred for 24 hours at room temperature, then diluted to 5 ml with dichloromethane, washed three times with 10 ml of water and four times with 10 ml of a saturated sodiumcarbonate solution.

After drying of the mixture over sodiumsulphate and boiling down, it was demonstrated with ESR that the dendrimer contained no proxyl radicals. This shows that the proxyl radical is not bonded chemically to the amino acid.

### Experiment V: Blank experiment B.

21 mg of proxylcarboxylic acid (proxyl radical) (0.12 mmol) was added to a mixture of 10 mg (13 µmol) of a NH₂-terminated polypropylamine dendrimer of the second generation (8-cascade: 1,4-diaminobutane[4]:(1-azabutylidene)⁴:propylamine) and 0.1 ml of triethylamine in 0.25 ml of dichloromethane. After stirring for 30 minutes at room temperature 37 mg (0.10 mmol) of the hydroxysuccinimide ester of N-BOC-L-phenylalanine was added. After stirring for one night at room temperature the reaction mixture was diluted to 50 ml with dichloromethane and next washed three times with 30 ml of water and three times with 30 ml of a saturated sodiumcarbonate solution. After drying over sodiumsulphate and boiling down, the yield was about 81%.

The ESR spectrum of the dendrimer dissolved in dichloromethane showed a trace of the proxyl radical.

The radical was completely removed by washing five times with 30 ml of a saturated sodiumcarbonate solution.

This shows that the proxyl radical is not bonded chemically to the dendrimer and that the cavities of a second generation polypropylamine dendrimer are too small to occlude proxyl radicals.

### Example VI: Occlusion of several proxylcarboxyl radicals per dendrimer.

58 mg of proxylcarboxylic acid (0.31 mmol) was added to a mixture of 22 mg (3 µmol) of a NH₂-terminated polypropylamine dendrimer of the fifth generation (64-cascade: 1,4-diaminobutane[4]:(1-azabutylidene)⁶⁰:propylamine) and 0.1 ml of triethylamine in 0.5 ml of dichloromethane. After stirring for 30 minutes 95 mg (0.26 mmol) of the hydroxysuccinimide ester of N-BOC-L-phenylalanine was added. The procedure as described in example III was repeated.

By means of ESR it was demonstrated that eight radicals per dendrimer molecule were occluded.

### Example VII: Occlusion of proxylcarboxylic acid in BOC-protected leucine.

The procedure as described in example III was repeated, using 0.53 mmol of the hydroxysuccinimide ester of BOC-protected leucine instead of the hydroxysuccinimide ester of BOC-phenylalanine. About 1 proxylcarboxylic acid radical per dendrimer molecule was occluded.

### Examples VIII-XI

The procedure as described in example I was repeated. In table 1 a survey is presented of the quantities applied and the active substances used.

**Table 1**

| Active substance | quantity of dendrimer (mg) | quantity of amino acid (mg) | quantity of active substance (mg) | load |
|---|---|---|---|---|
| 8. Reichardt E_{T}*,** probe | 38 | 127 | 11 | 2 |
| 9. Kosover* Z-probe | 25 | 82 | 3.3 | 0.7 |
| 10. Penicillin V | 39 | 129 | 39 | 2 |
| 11. Azlocillin (penicillin) | 119 | 391 | 18 | 0.3 |

| | | | | |
|---|---|---|---|---|
| * Probes for the determination of the polarity of a solvent using UV-spectroscopy | | | | |
| ** 2,6-diphenyl-4-(2,4,6-triphenylpyridinio)-phenolate | | | | |

### Examples XII-LXVIII

In the following examples various active substances were occluded in a dendrimer, such as radicals, colourants, merocyanine colourants, fluorescent compounds, chelating compounds, azanaphthol compounds (which become chiral through occlusion in the dendrimer), electron-deficient compounds and pyridine soaps.

In table 2 a survey is presented of the active substances used, the quantities of dendrimer, protected amino acid and active substance used and the quantity of active substance that was ocluded per dendrimer molecule.

### Standard procedure for loads up to 1.2 molecules of active substance per dendrimer molecule

X mg of an active substance was added to a mixture of Y mg of a NH₂-terminated polypropylamine dendrimer of the fifth generation (64-cascade: 1,4-diaminobutane[4]:(1-azabutylidene)⁶⁰:propylamine) and 0.1 ml of triethylamine in 10 ml of dichloromethane. After stirring for 30 minutes, 1 equivalent of the hydroxysuccinimide ester of N-BOC-L-phenylalanine was added per NH₂ group present in the dendrimer. The reaction mixture was stirred overnight. Then the reaction mixture was diluted to 50 ml with dichloromethane and subsequently washed three times with 30 ml of water and three times with 30 ml of a saturated sodiumcarbonate solution. After drying over sodiumsulphate and evaporation of the remaining liquid, the dendrimer containing active substance was isolated.

### Standard procedure for loads higher than 1.2 molecules of active substance her dendrimer molecule

In this case 0.5 ml of triethylamine and 0.5 ml of dichloromethane, respectively, were used instead of 0.1 ml of triethylamine and 10 ml of dichloromethane.

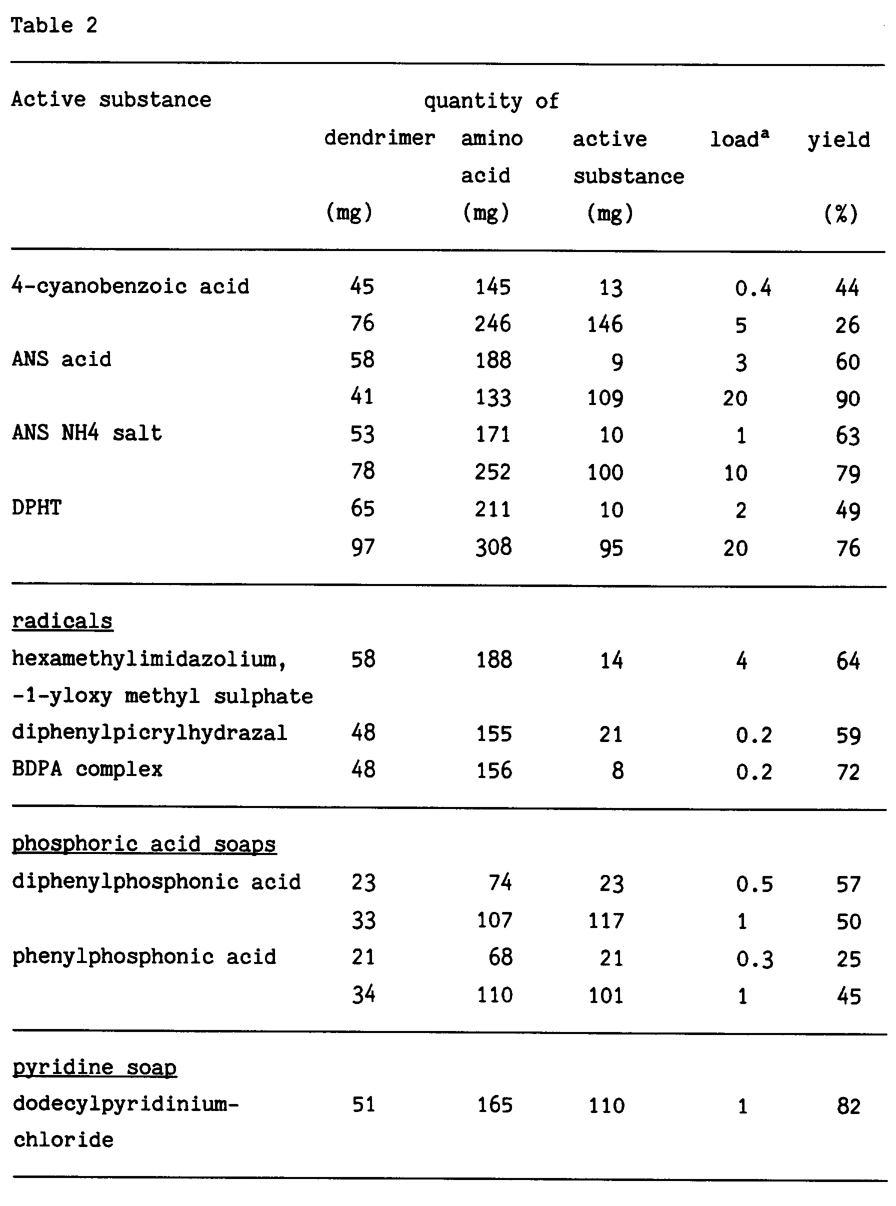

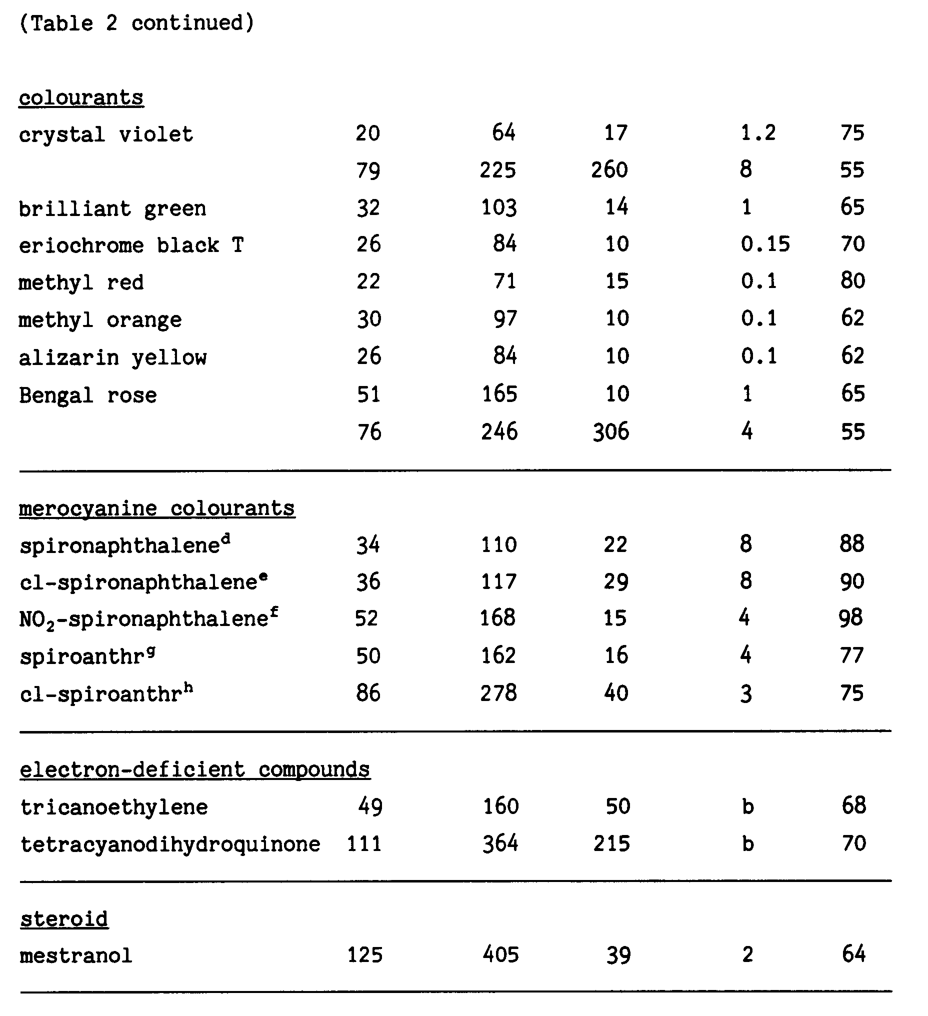

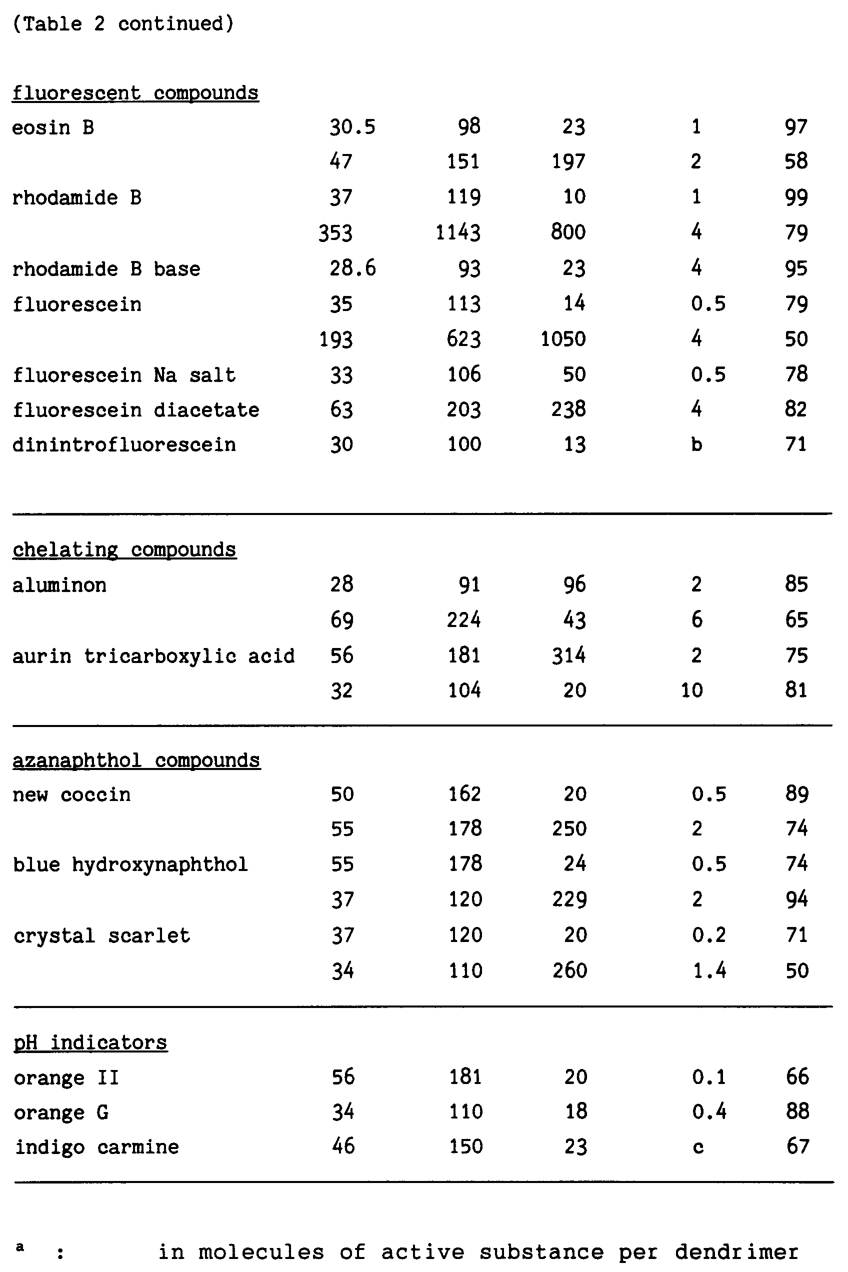

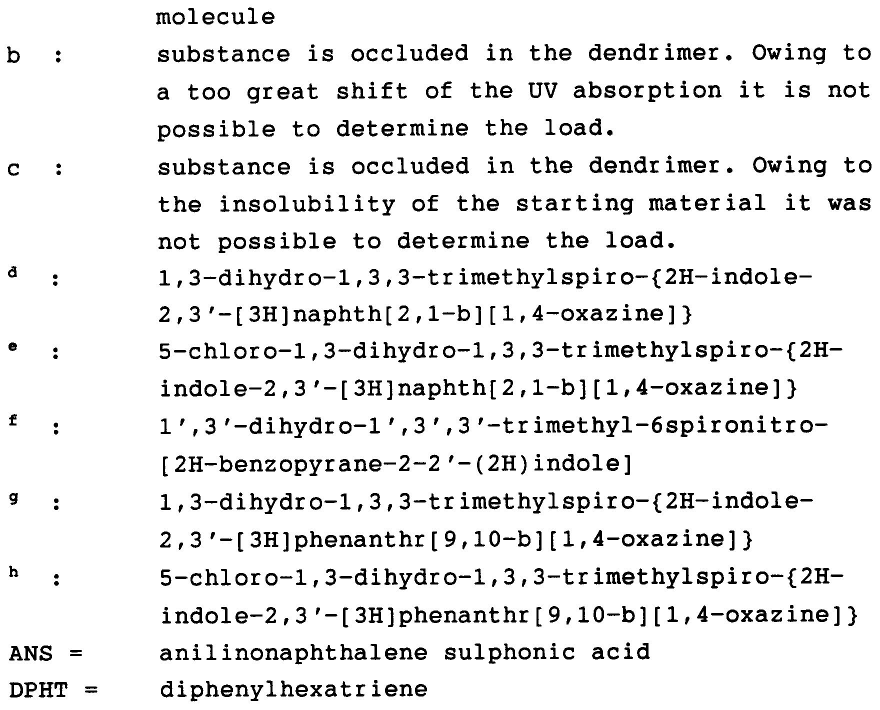

That the colourants listed in table 2 are actually occluded in the dendrimer can be concluded from the UV spectra. The UV spectrum signal of the N-containing colourants occluded in dendrimer shows a clear shift relative to the signal of the free colourant.

Bengal rose shows no signal in the UV spectrum and no fluorescence. However, after occlusion of the Bengal rose in the dendrimer a clear fluorescence spectrum was observed.

In order to demonstrate that eriochrome black T and alizarin yellow are actually occluded in the dendrimer, the following experiments were performed.

A sample flask was filled with eriochrome black T dissolved in acetonitrile, which resulted in a reddish brown solution. A second sample flask was filled with a mixture of acetonitrile and dendrimer containing eriochrome black. As the dendrimer does not dissolve in acetonitrile, the acetonitrile solution remained colourless. The colourant was observed in the form of black spots on the glass surface. The solution remained colourless even after a 2-hour ultrasonic treatment and 6 weeks' rest. This shows that the colourant is actually occluded in the dendrimer and is not released out of a dendrimer the terminal groups of which are provided with phenylalanine protected with a BOC group.

### Experiment LXIX: Blank experiment C.

By means of the example below it was demonstrated that the active substance is not bonded chemically to the amino acid.

To that end a mixture of 200 mg of the hydroxysuccinimide ester of N-BOC-L-phenylalanine, 100 mg of alizarin yellow and 10 ml of dichloromethane was stirred for 4 days at room temperature. Next, 50 mg of an amine-terminated second-generation polypropylamine dendrimer (8-cascade: 1,4-diaminobutane[4]:(1-azabutylidene)⁴:propylamine) was added to the mixture and stirred for a night at room temperature. The mixture was diluted to 50 ml with dichloromethane and next washed six times with 30 ml of a saturated sodiumcarbonate solution. After drying in the presence of sodiumsulphate and evaporation the dendrimer was isolated. The yield was 63%. The quantity of occluded alizarin yellow was negligibly small: 0.001 molecules per dendrimer molecule. This shows that the alizarin yellow is not bonded chemically to the amino acid.

Subsequently the dendrimer was dissolved in dichloromethane and again washed four times with 30 ml of a saturated sodiumcarbonate solution. After drying of the solution over sodiumsulphate and boiling down, the quantity of occluded alizarin yellow was 0.0001 molecules per dendrimer molecule.

### Example LXX-LXXI: Occlusion of metal salts.

50 mg of CuCl₂ and FeCl₃, respectively, was added to 86 mg of a NH₂-terminated polypropylamine dendrimer of the fifth generation (64-cascade: 1,4-diaminobutane[4]:(1-azabutylidene)⁶⁰:propylamine) and 0.1 ml of triethylamine in 10 ml of dichloromethane. After stirring for 30 minutes 310 mg of the hydroxysuccinimide ester of N-BOC-L-phenylalanine was added. The reaction mixture was stirred overnight. Next, the reaction mixture was diluted to 150 ml with dichloromethane and successively washed six times with 100 ml of a saturated sodiumcarbonate solution. After drying over sodiumsulphate and evaporation of the remaining liquid, the dendrimer containing active substance was isolated. The load was in both cases 2 molecules of salt per dendrimer molecule.

### Example LXXII and LXXIII

Example I was repeated in the presence of 38 mg of a polypropylamine dendrimer of the sixth generation (128-Cascade:1,4-diaminobutane[4]:(1-azabutylidene)¹²⁴:propylamine), 100 mg of the hydroxysuccinimidic ester of t-BOC-alanine and 227 mg of dinitrobenzoic acid. Approximately 8 molecules of nitrobenzoic acid were occluded in the dendrimer. Approximately 70% of the terminal amine groups of the dendrimer were modified with t-BOC-alanine.

Example I was repeated in teh presence of 134 mg of a polypropylamine dendrimer of the fourth generation (32-Cascade:1,4-diaminobutane[4]:(1-azabutylidene)²⁸:propylamine), 490 mg of the hydroxysuccinimidic ester of t-BOC-tryptophane and 286 mg of Bengale rose. Approximately 2 molecules of Bengale pink were occluded in the dendrimer. Approximately 71% of the terminal groups of the dendrimer were modified with t-BOC-tryptophane.

### Example LXXIV: Occlusion and selective release of two different active substances.

Example I was repated in the presence of 99 mg of a polypropylamine dendrimer of the fith generation (64-Cascade:1,4-diaminobutane[4]:(1-azabutylidene)⁶⁰:propylamine), 278 mg of the hydroxysuccinimidic ester of t-BOC-valine and 254 mg of dinitrobenzoic acid (active substance I) and 236 mg of Bengale rose (active substance II). Respectively 10 molecules of dinitronbenzoic aicd and 4 molecules of Bengale rose were occluded in the dendrimer.

After refluxing for 2 hours in formic acid and dialysis for 2 days in chloroform, the load of Bengale rose was 4 molecules per dendrimer molecule. Dinitrobenzoic acid was completely removed from the dendrimer.

After refluxing for 2 hours in hydrochloric acid and overnight dialysis is water, the load of Bengale pink was 0.005 molecules per dendrimer molecule. The dendrimer was recovered as a fifth genegration amine terminated dendrimer. All t-BOC-valine groups could be removed from the dendrimer.

### Example LXXV:

Example I was repeate din the presence of 99 mg of a polypropylamine dendrimer of the fith generation (64-Cascade:1,4-diaminobutane[4]:(1-azabutylidene)⁶⁰:propylamine), 278 mg of the hydroxyusccinimidic ester of t-BOC-valine and 75 mg of nitrophenol (active substance I) and 25 methylviolet 3 RAX (Aldrich) (active substance II) and a number of different blocking agents as shown in table 3a.

**TABLE 3a**

| blocking agent | mg pa | mg ester | Y(%) | load (I) | load (II) |
|---|---|---|---|---|---|
| N-FMOC-β-butyl-aspartic-hydroxysuccinimidic ester* | 51 | 236 | 40 | 8 | 8,2 |
| N-FMOC-O-t-butyl-L-serine-3,4-dihydro-4-oxo-1,2,3-benztriazool | 48 | 227 | 82 | 4 | 3,9 |
| N-FMOC-L-phenyl-alanine-pentafluorphenylester | 90 | 447 | 52 | 8 | 7,6 |
| N-FMOC-S-trityl-L-cysteënepentafluorphenylester | 71 | 478 | 53 | 4 | 4,2 |
| N-FMOC-L-alaninepentafluorphenylester | 49 | 208 | 79 | 8 | 7,8 |

| | | | | | |
|---|---|---|---|---|---|
| * After working up, 50% of the FMOC seemed to be lost. | | | | | |

Active substance I was electively released by first stirring the conjugate with a 20% solution of piperidine in dimethylformamide followed by 6 times dialysis in ethanol. The loads of respectively nitrophenol (I) and methylviolet 3 RAX (II) are shown in table 3b.

**TABLE 3b**

| blocking agent | load (I) | load (II) |
|---|---|---|
| N-FMOC-β-t-butyl-aspartic-hydroxysuccinimidic-ester | 0 | 3,9 |
| N-FMOC-O-t-butyl-L-serine-3,4-dihydro-4-oxo-1,2,3-benztriazool | 0 | 3,7 |
| N-FMOC-L-phenylalanine-pentafluorphenylester | 0 | 7,3 |
| N-FMOC-S-trityl-L-cysteëne-pentafluorphenylester | 0 | 4,0 |
| N-FMOC-L-alanine-pentafluorphenylester | 0 | 0,05 |

Then, the conjugate was boiled for two hours in a solution of 6N hyrochloric acid, the solvent was evaporated and the remaining solution was neutralised. The conjugate was then subjected succesively to a dialysis in ethanol and a dialysis in water. The loads of respectively nitrophenol (I) and methylviolet 3 RAX (II) are shown in tabel 3c.

**TABLE 3c**

| blocking agent | load (I) | load (II) |
|---|---|---|
| N-FMOC-β-t-butyl-aspartic-hydroxysuccinimide-ester | 0 | 0,01 |
| N-FMOC-O-t-butyl-L-serine-3,4-dihydro-4-oxo-1,2,3-benztriazool | 0 | 0,02 |
| N-FMOC-L-phenylalanine pentafluorphenylester | 0 | 0,02 |
| N-FMOC-S-trityl-L-cysteëne-pentafluorphenylester | 0 | 0,01 |
| N-FMOC-L-alanine pentafluorphenylester | 0 | 0,02 |

From these examples it can be seen that active substances with different dimensions can selectively be released from the dendrimer.

In case N-FMOC-L-alanine pentafluorfenylester is used as a blocking agent, the active substances I and II are released after a treatment with piperidine followed by a dialysis in respectively dimethylformamide and ethanol.

## Claims

1. Composition consisting of a dendrimer and an active substance occluded in the dendrimer wherein the dendrimer has terminal groups, characterized in that the terminal groups of the dendrimer are provided with blocking agents.

2. Composition according to claim 1, characterized in that the blocking agents are at least partly provided with protective groups.

3. Composition according to claims 1-2, characterized in that the blocking agents are chosen from the group comprising branched and non-branched Michael acceptors, active esters which also comprise an ether and/or thiol group, amino acids, isocyanates, aziridines, acid chlorides, anhydrides, aldehydes, ketones, acrylates, chiral epoxides, bislactide.

4. Composition according to any one of claims 1-3, characterized in that the blocking agent is an amino acid.

5. Composition according to any one of claims 2-4, characterized in that the protective group is chosen from the range comprising hydrolyzable esters, ethers, thiol groups, sulphonic acid groups, trityl, silyl, BOC, FMOC, DBD-TMOC, Z, TEOC, AdMOC, STABASE and benzoSTABASE groups.

6. Composition according to claim 5, characterized in that the protective group is a BOC group.

7. Composition according to any one of claims 2-6, characterized in that at least 30% of the terminal groups are provided with a blocking agent with a protective group.

8. Composition according to claim 7, characterized in that at least 70% of the terminal groups of the dendrimer are provided with a blocking agent with a protective group.

9. Composition according to any one of claims 1-8, characterized in that the composition contains several different active substances.

10. Composition according to any one of claims 1-9, characterized in that the active substance belongs to the range of pharmaceutical compounds, agrochemicals, materials used in personal care products, cosmetic products, food additives, aspartame, additives for engineering plastics, signal absorbers, signal generators, chelating compounds, radicals, soaps, colourants, radio active labelled compounds, metals, metal compounds, radionuclides, electron deficient and non-metallic electron rich compounds and precursors thereof.

11. Composition according to any one of claims 1-10, characterized in that the dendrimer is a polypropylamine dendrimer.

12. Composition according to claim 11, characterized in that the dendrimer is a dendrimer of generation 4 or higher.

13. Process for the preparation of a composition according to any one of claims 1-12, characterized in that an amount of an active substance to be occluded is added to a reaction mixture containing dendrimers and simultaneously or subsequently an amount of blocking agent provided with protective groups is added.

14. Process for releasing an active substance from a composition according to any one of claims 1-12, characterized in that the active substance is released by splitting off or modifying the blocking agent.

15. Process for releasing an active substance from a composition according to any one of claims 2-12, characterized in that the active substance is released by splitting off the protective group.

16. Process according to claim 15, characterized in that the active substance is released by first splitting off the protective group and then splitting off the blocking agent.

17. Process according to any one of claims 13-16, characterized in that the protective group and/or the blocking agent are removed by means of a chemical reaction, hydrolysis in a neutral, acidic or basic medium, hydrogenation, a reaction under the influence of heat, a photochemical reaction, reaction in the presence of fluoride, a retro Michael reaction.

18. Process according to claim 14, characterized in that the active substance is released by changing the physical interactions within the dendrimer provided with blocking agents.

19. Composition, process for the preparation of the composition and process for releasing an active substance as described and elucidated by means of the examples.
